# EUROPEAN PATENT SPECIFICATION

(11) **EP 4 473 983 B1**
(45) Date of publication and mention of the grant of the patent: **25.03.2026**
(21) Application number: 23841483.3
(22) Date of filing: 17.04.2023
(51) Int. Cl.: A61L 27/06, A61L 2/04, C25D 11/00, A61C 8/00

(54) **METHOD FOR PASSIVATING SURGICAL IMPLANTS COMPRISING TITANIUM AND SURGICAL IMPLANT OBTAINED**
VERFAHREN ZUR PASSIVIERUNG VON CHIRURGISCHEN IMPLANTATEN MIT TITAN UND HERGESTELLTES CHIRURGISCHES IMPLANTAT
PROCÉDÉ DE PASSIVATION D'IMPLANTS CHIRURGICAUX QUI RENFERMENT DU TITANE ET IMPLANT CHIRURGICAL OBTENU

(43) Date of publication of application: 11.12.2024
(73) Proprietor: Safe Titanium, S.L., 08017 Barcelona (ES)
(72) Inventor: GIL MUR, Javier, 08017 BARCELONA (BARCELONA) (ES); FERNANDEZ FAIREN, Mariano, 08017 BARCELONA (BARCELONA) (ES)
(74) Representative: Durán-Corretjer, S.L.P.
(86) International application number: PCT/ES2023/070238
(87) International publication number: WO 2024/218409

(56) References cited:
- CN-A- 102 552 044
- CN-A- 102 978 677
- CN-A- 110 338 921
- CN-A- 111 455 389
- US-A1- 2015 216 630
- DOE YUJIRO ET AL: "Titanium surface treatment by calcium modification with acid-etching promotes osteogenic activity and stability of dental implants", MATERIALIA, vol. 12, 18 June 2020 (2020-06-18), pages 100801, XP093285807, ISSN: 2589-1529, DOI: 10.1016/j.mtla.2020.100801
- CRUZ NUNO ET AL: "Relevant Aspects of Piranha Passivation in Ti6Al4V Alloy Dental Meshes", COATINGS, vol. 12, no. 2, 27 January 2022 (2022-01-27), CH, pages 154 - 1, XP093227242, ISSN: 2079-6412, DOI: 10.3390/coatings12020154
- CRUZ NUNO, GIL JAVIER, PUNSET MIQUEL, MANERO JOSÉ MARÍA, TONDELA JOÃO PAULO, VERDEGUER PABLO, APARICIO CONRADO, RÚPEREZ ELISA: "Relevant Aspects of Piranha Passivation in Ti6Al4V Alloy Dental Meshes", COATINGS, vol. 12, no. 2, 27 January 2022 (2022-01-27), CH , pages 154-1 - 154-17, XP093227242, ISSN: 2079-6412, DOI: 10.3390/coatings12020154
- PADULLES-GASPAR ESTEBAN, PADULLES-ROIG ESTEBAN, CABANES GUILLERMO, PÉREZ ROMÁN A., GIL JAVIER, BOSCH BEGOÑA M.: "Effects of Hypochlorous Acid and Hydrogen Peroxide Treatment on Bacterial Disinfection Treatments in Implantoplasty Procedures", MATERIALS, vol. 16, no. 8, CH , pages 2953 - 2953-13, XP093227244, ISSN: 1996-1944, DOI: 10.3390/ma16082953

## Description

The present invention relates to the field of traumatology and orthopaedics and, more particularly, to a method for the passivation treatment of a surgical implant made of titanium. The present invention also relates to a titanium surgical implant whose surface oxide layer has very low bacterial adherence properties and, in addition, is capable of facilitating bone generation.

In recent years, implantology, like other areas of odontostomatology, has undergone constant changes in its development due to the emergence of new technologies. This transformation can be attributed, in part, to the emergence of new implant designs, surface treatments and better understanding of the biology of osseointegration.

Such implants in the mouth are in basic terms a collection of different metals and alloys placed in an electrolyte: saliva. This system produces corrosion, i.e. the degradation or destruction of the metals by reaction with their environment. When a metal is in an aqueous solution, such as saliva, there is, on the one hand, a metallic phase made up of M²⁺ ions forming a crystal lattice and conduction electrons, which are free electrons that can move, and on the other hand, a liquid aqueous phase called an electrolyte, which gives rise to corrosion.

An alloy is a mixture of two or more metals. In dentistry, alloys generally contain at least four metals and often more than six, and can thus be considered to be metallurgically complex. The high cost of gold alloys led to the use and development of other alloys of different compositions, with the aim of achieving good mechanical properties and a good cost-effectiveness ratio, but their biocompatibility and resistance to corrosion continue to be a source of controversy, since, as has been shown in various studies, the combination of non-noble alloys (or of alloys with low resistance to corrosion) with titanium can give rise to galvanic corrosion phenomena, even resulting in the failure of the implant.

Titanium is one of the metals used for metal implants. It is a light metal (4.5 g/cm³), weighing 45% less than steel (7.8 g/cm³) and only 60% more than aluminium (2.7 g/cm³). This advantage is accompanied by excellent toughness, fatigue strength and corrosion resistance, as it withstands a very wide range of corrosive agents, high pressures and higher temperatures than many other metals, and is of low weight. Its high strength-to-weight ratio affords titanium-based alloys better performance than other metals and engineering alloys over a wide temperature range. Another advantage of titanium in biomedical applications is its modulus of elasticity of 110 GPa, which is half that of other alloys proposed for implants, making it more elastically compatible with bone, which has an elastic modulus of 20 GPa. This optimizes the load transfer process between implant and bone.

Knowledge of the physicochemical properties of titanium and its alloys involves the exhaustive study of the properties of the titanium oxide layer that grows on it naturally and spontaneously, on contact with air and other media, which is known as passivation. This layer protects the metal against an uncontrolled increase in its oxidation, undesirable chemical and biological reactions, and corrosion. As a consequence, chemical and biological agents do not interact directly with the metal, but with this stable titanium oxide layer.

A number of different stoichiometries of titanium oxides can be identified on the surface of titanium (Ti₃O, Ti₂O, Ti₃O₂, TiO, Ti₂O₃, Ti₃O₅ and TiO₂). The most stable of these is TiO₂, with titanium in its preferred oxidation state: +IV. Table 1 details the surface properties of titanium and its most stable oxide, which have been shown to influence the interaction between the metal and proteins and cells.

**Table 1. Selected physicochemical properties of titanium and its oxide**

| | |
|---|---|
| Most stable oxide | TiO₂ |
| Isoelectric point | 3.5-6.7 |
| Charge at pH = 7 | Negative |
| Dielectric constant of the oxide | 86-170 |
| Solubility at pH = 7 [mol/l] | 3·10⁻⁶ |
| Charge of the dissolved species | 0 |
| Water contact angle | 54 |
| Free surface energy (FSE) [mJ/m²] | 50.0 |
| Polar component of the FSE [mJ/m²] | 31.7 |
| Dispersive component of the FSE [mJ/m²] | 18.3 |

The advantages of titanium may be listed in the following manner: (a) the highly protective nature of the oxide layer, which is generally only a few nanometres thick, is a consequence of its natural integrity and chemical stability over a wide range of pH values, electrolytes and bodily fluids; (b) the surface titanium oxide quickly repassivates after a local loss of passivation, for instance resulting from mechanical wear; (c) the low solubility of hydrated titanium oxides, together with the even lower tendency to form charged titanium compounds, are very relevant aspects for the biocompatibility of titanium; (d) a certain physicochemical similarity between the clean surface of titanium oxide and water can be assumed as a consequence of the extensive hydroxylation/hydration of the oxide and its moderate hydrophilicity. This leads to a certain amount of surface interaction with the water shell that forms around biomolecules such as proteins; (e) the dielectric constant of the oxide is similar to that of water. As is the case with aqueous fluids, this results in reduced polarization effects and shielding of electrostatic forces between charged particles; (f) the low surface electrical charge, since the isoelectric point of titanium oxide is only slightly below physiological pH, is believed to reduce the risk of strong interactions becoming established between the titanium surface and the charge domains of proteins; (g) the "natural ability" to form calcium-carbonate-phosphate layers on the surface of titanium oxide through specific chemical exchange methods with the constituents of bodily fluids (blood, interstitial fluid), leads, after some time *in vivo*, to modification of the synthetic material/biological material interface, via the formation of a layer a few nanometres thick.

In addition, other properties such as residual stresses, grain size, degree of toughness, or magnetic properties can also have an influence on the response of the manufactured titanium implant.

Titanium alloys used in orthopaedic surgery are corrosion resistant and compatible with the human body due to the spontaneously formed titanium oxide film on the surface, which is only a few nanometres thick. However, the use of more than one type of alloy or metal in the same patient for different dental treatments in an aggressive salivary electrolyte medium can facilitate corrosion.

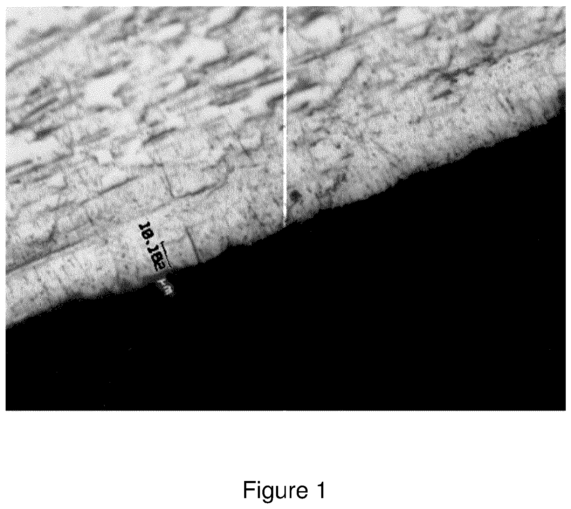

In addition, the stability and thinness of the oxide layer may accelerate the degradation process of metallic biomaterials. Another important aspect to take into account is the release of metal ions into the physiological environment, which has become a concern for health authorities as it can cause health problems for patients, notably dental patients who have incorporated metallic materials of different chemical nature in their mouths. It is known that metals in sufficient concentration are toxic and can cause inflammatory or allergic processes, genetic mutations or carcinogenic processes. In order to minimize the corrosion of the implants and the release of metal ions into the physiological environment and to clean the remains of the manufacturing methods, an anodizing treatment is used, which consists in making the titanium oxide layer grow in a controlled manner in order to obtain a tough, homogeneous, highly impermeable and stable layer. This method of modifying the oxide layer is also called the passivation method, as it makes the prosthesis passive to chemical attack. Figure 1 shows the homogeneous and stable titanium oxide layer, not showing any cracks that could indicate fragility: in this case the titanium was treated with a solution of nitric acid with hydrofluoric acid for 30 seconds.

The passivation or anodizing method can be chemical or electrochemical, and is performed by immersing the titanium in an oxidizing acidic medium. Oxidizing acids such as nitric, sulfuric, hydrochloric and other acids can be used at a certain concentration, which causes the oxide layer formed on the surface to grow. This method can be assisted by the passage of an electric current in an electrochemical cell which produces stable titanium oxide layers.

US Patent application US 2015/216630 A1 discloses a titanium implant featuring a surface treated by sulfuric and hydrochloric acid etching, resulting in a nanoscale topography. This topography includes nanopits created by immersing the implant in a solution containing hydrogen peroxide and a basic agent. The nanoscale roughness is visible in the 1-100 nanometer range.

Chinese Patent application CN 111 455 389 A discloses titanium implants sandblasted with white corundum sand, etched in a mixture of sulfuric acid and hydrochloric acid, and subsequently soaked in hydrogen peroxide. The resulting hydrophilic titanium implant has nanoparticles or nanopores below 100 nm.

Doe Yujiro et al. (Materialia, Vol. 12, 2020, DOI: 10.1016/j.mtla.2020.100801) disclose a pure titanium implant surface treated by acid-etching with a mixture of hydrochloric and sulfuric acids, followed by cleaning and drying. The implant is then immersed in an aqueous calcium chloride solution with ozone, and stored in a an aqueous calcium chloride solution without ozone.

Cruz Nuno et al. (Coatings, Vol. 12, Article 154, 2022, DOI: 10.3390/coatings12020154) disclose titanium alloy (Ti6Al4V) meshes treated by immersion in a solution containing sulfuric acid, hydrochloric acid, and hydrogen peroxide, followed by cleaning.

In the light of the foregoing, there is still a need to obtain a method for the passivation of a surgical implant comprising titanium, which topographically produces a passivation layer which displays very low bacterial adhesion and which, in addition, can release divalent cations that are capable of facilitating bone generation.

Thus, in a first aspect, the present invention discloses a method for the passivation of a surgical implant comprising titanium, by means of which it is possible to obtain a passivating layer on said implant which has the abovementioned characteristics.

In the present invention, the term "passivation", "passivating" or "anodizing" are used interchangeably to refer to the formation of a relatively inert titanium oxide film on the surface of the implant comprising titanium, which masks it against the action of external agents. The passivating layer or film does not allow such external agents to interact, and as such the chemical or electrochemical reaction is reduced or completely prevented.

Many advantages exist as regards implants treated by means of the method of the present invention, such as: (1) cleaning of both organic and inorganic residues from the surface of the biomaterial; (2) improved corrosion resistance of the implant; (3) decreased release of titanium ions into the physiological environment; and (4) increased surface hardness and also wear resistance.

In the present invention, the term "surgical implant" refers to an implantable medical device which has a therapeutic purpose, which totally or partially replaces a body structure or a physiological function that is defective or anomalous, or which has a diagnostic purpose.

Thus, in a first aspect, the present invention discloses a method for the passivation of surgical implants comprising titanium, characterized in that it comprises the steps of:
a) immersing said surgical implant in a solution comprising hydrochloric acid, sulfuric acid and hydrogen peroxide;
b) performing at least two washes with an aqueous solution; and
c) performing at least one wash with an alcohol.

According to the invention, the solution of step a) also comprises divalent cations. Said J divalent cations are calcium (Ca²⁺) and/or magnesium (Mg²⁺).

Preferably, step a) has a duration of between 30 min and 4 hours, more preferably between 45 min and 3 hours, even more preferably between 1 and 2 hours.

Preferably, the hydrochloric acid has an initial concentration of between 10% and 30% (v/v), more preferably, between 12% and 25% (v/v). Even more preferably, the hydrochloric acid concentration is 20% (v/v).

Preferably, the sulfuric acid has an initial concentration of between 89% and 99% (v/v), more preferably, between 93% and 99% (v/v). Even more preferably, the sulfuric acid concentration is 96% (v/v).

Preferably, the hydrogen peroxide has an initial concentration of between 20% and 40% (v/v), more preferably, between 25% and 35% (v/v). Even more preferably, the hydrogen peroxide concentration is 30% (v/v).

Preferably, the ratio of hydrochloric acid and sulfuric acid to hydrogen peroxide is between 40% and 60% for each by volume.

Preferably, the aqueous solution of step b) is distilled water, deionized water or a mixture of the two.

Preferably, the alcohol of step c) is methyl alcohol (methanol).

Preferably, after washing with methyl alcohol, washing with acetone is performed, optionally followed by drying with a stream of hot air.

Preferably, the washes of steps b) and c) have a duration of between 1 min and 2 min.

Preferably, the washes are performed in an ultrasonic bath.

Furthermore, the present disclosure describes a surgical implant comprising titanium which displays very low bacterial adherence, characterized in that it comprises a surface layer of titanium oxide with a nanoporosity of between 50 nm and 500 nm. More preferably, the nanoporosity is between 60 nm and 400 nm. More preferably, the nanoporosity is between 70 nm and 220 nm. More preferably, the nanoporosity is between 80 nm and 210 nm. Even more preferably, the nanoporosity is between 90 nm and 200 nm.

Preferably, said surgical implant has been previously treated by means of the passivation method of the present invention.

Finally, the present invention discloses the use of a solution comprising hydrochloric acid, sulfuric acid, hydrogen peroxide and divalent cations for the passivation of surgical implants comprising titanium, wherein said divalent cations are calcium (Ca²⁺) and/or magnesium (Mg²⁺).

For ease of comprehension, the present invention is described in greater detail hereinbelow, with reference to the attached figures, which are presented by way of example, and with reference to illustrative but non-limiting examples.
Figure 1 is a photograph illustrating a titanium surface showing the passivation layer (titanium dioxide, TiO₂) formed after using a composition according to the prior art, i.e. with a solution of 15% (v/v) nitric acid and 30% (v/v) hydrofluoric acid for 30 seconds.

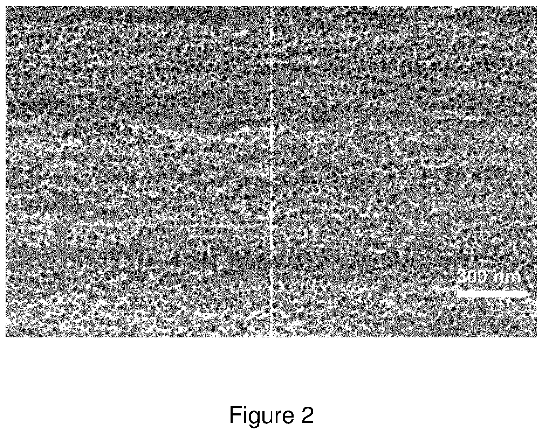

Figure 2 is a photograph illustrating a titanium surface after being treated with a solution according to the present invention.
Figure 3 shows scanning electron microscopy (SEM) images illustrated in the top line and fluorescence microscopy images illustrated in the bottom line of *Pseudomonas aeruginosa* (*P. aeruginosa*) stained using a live (green)/dead (red) bacterial viability kit.
Figure 4 shows scanning electron microscopy (SEM) images illustrated on the top line and fluorescence microscopy images illustrated on the bottom line of *Streptococcus sanguinis* (*S. sanguinis*) stained using a live (green)/dead (red) bacterial viability kit.
Figure 5 is a photograph illustrating a titanium mesh nanostructure obtained after being treated by means of the method of the present invention.
Figure 6 presents scanning electron microscopy (SEM) images showing histologies in dental implants placed in minipig pigs at different implantation times.
Figure 7 is a graph representing values of bone-to-implant contact (BIC) osseointegration percentages and total bone area (TBA).

In the following, the present invention is illustrated by means of examples, which do not constitute a limitation thereof.

### EXAMPLES

### Example 1: Passivation treatment of implants comprising titanium.

To perform the study, implants comprising titanium were subjected to three different treatments: (1) control (untreated), (2) with hydrochloric acid (HCl) at a concentration of 1 M and (3) with the solution of the present invention (hydrochloric acid at a concentration of 20% (v/v), concentrated 96% (v/v) sulfuric acid and hydrogen peroxide at a concentration of 30% (v/v)). The ratio of hydrochloric acid and sulfuric acid to hydrogen peroxide is 50% each by volume.

Example 2: Quantitative analyses of the bacterial adhesion test performed with the Gram-negative strain *P*. *aeruginosa* and the Gram-positive strain *S. sanguinis.*

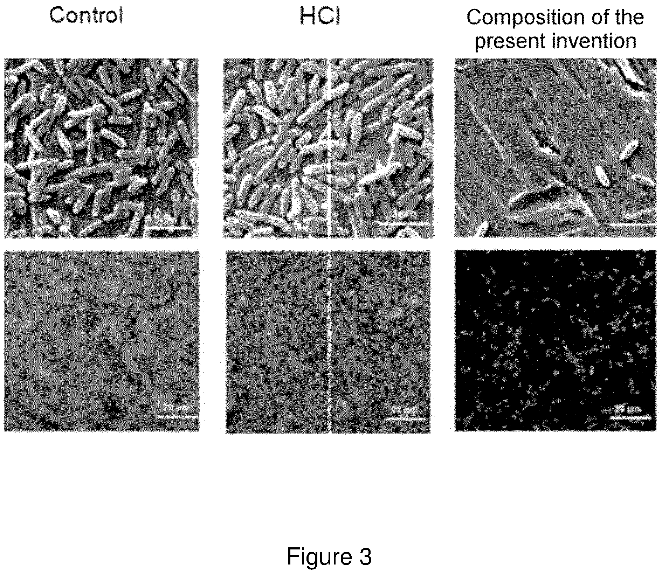

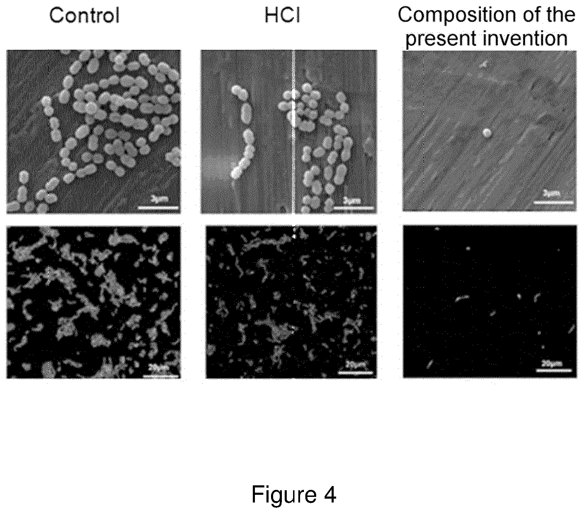

The quantitative analyses of the bacterial adhesion test performed with the Gram-negative strain *P*. *aeruginosa* and the Gram-positive *S. sanguinis* show that there are no significant differences in the number of bacteria adhered to the upper side of the control and hydrochloric acid-treated surfaces, but that there were differences with the meshes treated with the solution of the present invention (Table 2). In fact, for both bacterial strains, the titanium alloy surfaces treated by means of the method of the present invention drastically reduced (by at least an order of magnitude) bacterial adhesion compared to all other groups. Bacteria adhering to the differently treated surfaces can be seen in Figure 3 for *P. aeruginosa* and Figure 4 for *S. sanguinis,* which corroborates the quantification differences assessed for bacterial adhesion. The live/dead images revealed that the differences in bacterial numbers were mainly related to the prevention of bacterial colonization of the surfaces treated by means of the method of the present invention, as virtually none of the bacteria remaining on the surfaces had compromised membranes (red colour).

**Table 2. Quantitative analysis of the number of P. aeruginosa and S. sanguinis bacteria adhering to surfaces of grade V titanium alloys with different passivation treatments.**

| | *P. aeruginosa* (number of bacteria/mm²) | *S. sanguinis* (number of bacteria/mm²) |
|---|---|---|
| Control | 7.02x10⁵± 0.52 x10⁵ | 3.52x10⁵± 0.48 x10⁵ |
| HCl | 5.75 x10⁵±0.33 x10⁵ | 2.25 x10⁵±0.13 x10⁵ |
| Composition of the present invention | 1.23 x10⁴± 0.02x10⁴ | 5.03 x10³± 0.10x10³ |

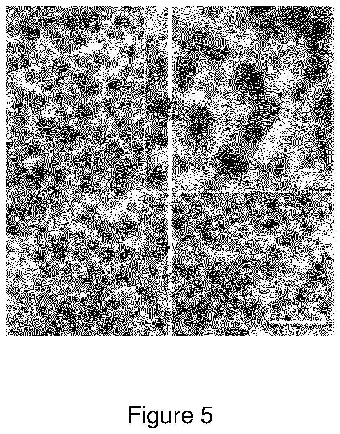

The characteristic nanotexture resulting from the passivation treatment of titanium alloy meshes with the solution of the present invention (Figure 5) was a relevant surface property achieved with the treatment of the present invention compared to the treatment with HCl. The meshes treated with the solution of the present invention showed a sub-microtexture with overlapping nanoporosity between 90 nm and 200 nm. This surface topography was homogeneous and without cracks, suggesting good toughness of the oxide layer formed. The presence of grooves on the treated surfaces could be related to a preferential etching method in areas with high internal energy, such as grain boundaries, dislocation stacking or other metallurgical or crystallographic singularities.

In the experiments performed, the inventors evaluated the effects of the passivation treatment on bacterial adhesion, as infection is a growing concern for dental meshes. Titanium alloy surfaces treated with the solution of the present invention were found to prevent bacterial adhesion significantly more effectively than untreated and HCl-treated surfaces.

Furthermore, divalent cations such as calcium and/or magnesium are added to the acidic solution, which impregnate the passivation layer and their release on physiological contact causes the rapid migration of osteoblast precursor proteins. This release of cations causes the rapid adhesion of osteoblasts, subsequent cell proliferation and differentiation, favouring a certain level of osteoconduction, increasing the speed of osseointegration and the level of bone-to-implant contact (BIC).

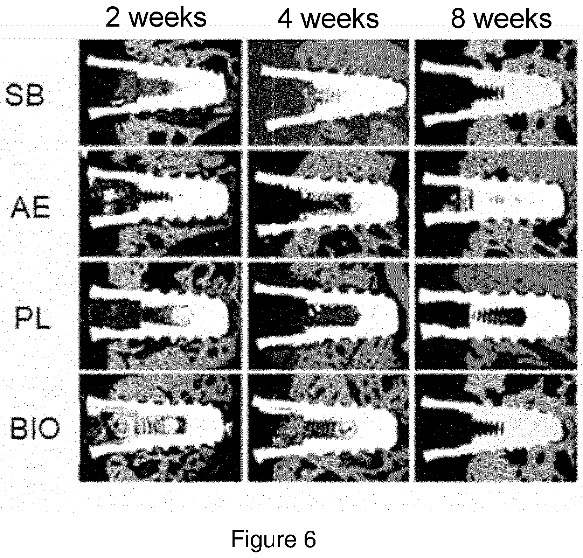

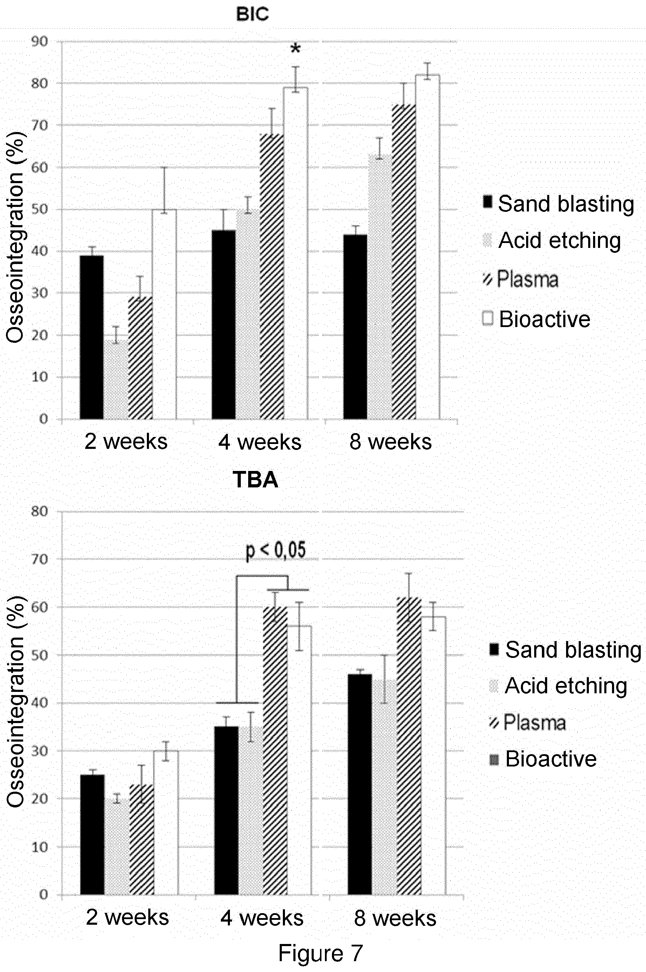

Figure 6 shows the histologies of dental implants that release calcium (BIO) with respect to sand blasting (SB), acid etching (AE) or plasma treatment (PL). The higher bone-to-implant contact (BIC) index and total bone area (TBA) for divalent cation-treated implants may be seen in Figure 7.

## Claims

1. Method for the passivation of a surgical implant comprising titanium, **characterized in that** it comprises the steps of:
a) immersing said surgical implant in a solution comprising hydrochloric acid, sulfuric acid and hydrogen peroxide;
b) performing at least two washes with an aqueous solution; and
c) performing at least one wash with an alcohol;
**characterized in that** the solution of step a) also comprises divalent cations, wherein said divalent cations are calcium (Ca²⁺) and/or magnesium (Mg²⁺).

2. Method according to claim 1, **characterized in that** step a) has a duration of between 30 minutes and 4 hours.

3. Method according to claims 1-2, **characterized in that** said hydrochloric acid has an initial concentration of between 10% and 30% (v/v).

4. Method according to any one of the preceding claims, **characterized in that** the sulfuric acid has an initial concentration of between 90% and 99% (v/v).

5. Method according to any one of the preceding claims, **characterized in that** the hydrogen peroxide has an initial concentration of between 20% and 40% (v/v).

6. Method according to any one of the preceding claims, **characterized in that** the ratio of hydrochloric acid and sulfuric acid to hydrogen peroxide is between 40% and 60% for each by volume.

7. Method according to any one of the preceding claims, **characterized in that** the aqueous solution of step b) is distilled water.

8. Method according to any one of the preceding claims, **characterized in that** the alcohol of step c) is ethanol.

9. Method according to any one of the preceding claims, **characterized in that** the washes of steps b) and c) have a duration between 1 min and 2 min.

10. Method according to any one of the preceding claims, **characterized in that** the washes of steps b) and c) are performed in an ultrasonic bath.

11. Use of a solution comprising hydrochloric acid, sulfuric acid, hydrogen peroxide and divalent cations for the passivation of a surgical implant comprising titanium, wherein said divalent cations are calcium (Ca²⁺) and/or magnesium (Mg²⁺).

## Patentansprüche

1. Verfahren zur Passivierung eines chirurgischen Implantats, das Titan umfasst, **dadurch gekennzeichnet, dass** es die folgenden Schritte umfasst:
a) Eintauchen des chirurgischen Implantats in eine Lösung, die Salzsäure, Schwefelsäure und Wasserstoffperoxid enthält;
b) Durchführen von mindestens zwei Waschvorgängen mit einer wässrigen Lösung; und
c) Durchführen von mindestens einem Waschvorgang mit einem Alkohol;
**dadurch gekennzeichnet, dass** die Lösung aus Schritt a) auch zweiwertige Kationen enthält, wobei die zweiwertigen Kationen Calcium (Ca²⁺) und/oder Magnesium (Mg²⁺) sind.

2. Verfahren nach Anspruch 1, **dadurch gekennzeichnet, dass** Schritt a) eine Dauer zwischen 30 Minuten und 4 Stunden hat.

3. Verfahren nach den Ansprüchen 1-2, **dadurch gekennzeichnet, dass** die Salzsäure eine Anfangskonzentration zwischen 10 % und 30 % (v/v) hat.

4. Verfahren nach einem der vorstehenden Ansprüche, **dadurch gekennzeichnet, dass** die Schwefelsäure eine Anfangskonzentration zwischen 90 % und 99 % (v/v) aufweist.

5. Verfahren nach einem der vorstehenden Ansprüche, **dadurch gekennzeichnet, dass** das Wasserstoffperoxid eine Anfangskonzentration zwischen 20 % und 40 % (v/v) aufweist.

6. Verfahren nach einem der vorstehenden Ansprüche, **dadurch gekennzeichnet, dass** das Verhältnis von Salzsäure und Schwefelsäure zu Wasserstoffperoxid zwischen 40 % und 60% pro Volumen beträgt.

7. Verfahren nach einem der vorstehenden Ansprüche, **dadurch gekennzeichnet, dass** die wässrige Lösung in Schritt b) destilliertes Wasser ist.

8. Verfahren nach einem der vorstehenden Ansprüche, **dadurch gekennzeichnet, dass** der Alkohol aus Schritt c) Ethanol ist.

9. Verfahren nach einem der vorstehenden Ansprüche, **dadurch gekennzeichnet, dass** die Waschvorgänge aus den Schritten b) und c) eine Dauer zwischen 1 min und 2 min haben.

10. Verfahren nach einem der vorstehenden Ansprüche, **dadurch gekennzeichnet, dass** die Waschvorgänge der Schritte b) und c) in einem Ultraschallbad durchgeführt werden.

11. Verwendung einer Lösung, die Salzsäure, Schwefelsäure, Wasserstoffperoxid und zweiwertige Kationen zur Passivierung eines chirurgischen Implantats aus Titan, wobei die zweiwertigen Kationen Calcium (Ca²⁺) und/oder Magnesium (Mg²⁺) sind.

## Revendications

1. Procédé de passivation d'un implant chirurgical comprenant du titane, **caractérisé en ce qu'**il comprend les étapes consistant à :
a) immerger ledit implant chirurgical dans une solution comprenant de l'acide chlorhydrique, de l'acide sulfurique et du peroxyde d'hydrogène ;
b) effectuer au moins deux lavages avec une solution aqueuse ; et
c) effectuer au moins un lavage avec un alcool ;
**caractérisé en ce que** la solution de l'étape a) comprend également des cations divalents, lesdits cations divalents étant le calcium (Ca²⁺) et/ou le magnésium (Mg²⁺).

2. Procédé selon la revendication 1, **caractérisé en ce que** l'étape a) a une durée comprise entre 30 minutes et 4 heures.

3. Procédé selon les revendications 1 à 2, **caractérisé en ce que** ledit acide chlorhydrique a une concentration initiale comprise entre 10 % et 30 % (v/v).

4. Procédé selon l'une quelconque des revendications précédentes, **caractérisé en ce que** l'acide sulfurique a une concentration initiale comprise entre 90 % et 99 % (v/v).

5. Procédé selon l'une quelconque des revendications précédentes, **caractérisé en ce que** le peroxyde d'hydrogène a une concentration initiale comprise entre 20 % et 40 % (v/v).

6. Procédé selon l'une quelconque des revendications précédentes, **caractérisé en ce que** le rapport entre l'acide chlorhydrique et l'acide sulfurique et le peroxyde d'hydrogène est compris entre 40 % et 60 % pour chacun en volume.

7. Procédé selon l'une quelconque des revendications précédentes, **caractérisé en ce que** la solution aqueuse de l'étape b) est de l'eau distillée.

8. Procédé selon l'une quelconque des revendications précédentes, **caractérisé en ce que** l'alcool de l'étape c) est de l'éthanol.

9. Procédé selon l'une quelconque des revendications précédentes, **caractérisé en ce que** les lavages des étapes b) et c) ont une durée comprise entre 1 min et 2 min.

10. Procédé selon l'une quelconque des revendications précédentes, **caractérisé en ce que** les lavages des étapes b) et c) sont effectués dans un bain à ultrasons.

11. Utilisation d'une solution comprenant de l'acide chlorhydrique, de l'acide sulfurique, du peroxyde d'hydrogène et des cations divalents pour la passivation d'un implant chirurgical comprenant du titane, dans laquelle lesdits cations divalents sont le calcium (Ca²⁺) et/ou le magnésium (Mg²⁺).
